# EUROPÄISCHE PATENTANMELDUNG

(11) **EP 2 144 061 A1**
(43) Veröffentlichungstag der Anmeldung: **13.01.2010**
(21) Anmeldenummer: 08012622.0
(22) Anmeldetag: 11.07.2008
(51) Int. Cl.: G01N 33/49, C12Q 1/56, G01N 27/30, G01N 33/86

(54) **Kontinuierliches Verfahren zum Inline-Auftrag von Tensid auf beschichteter Sensorfolie**

(71) Anmelder: F. Hoffmann-La Roche AG, 4070 Basel (CH); Roche Diagnostics GmbH, 68305 Mannheim (DE)
(72) Erfinder: Moldt, Marc, 68163 Mannheim (DE); Klonig, Kurt, 67472 Esthal (DE); Nortmeyer, Christine, 68305 Mannheim (DE); Zimmer, Volker, Laumersheim 67299 (DE)
(74) Vertreter: Dey, Michael

(57) **Zusammenfassung**

Die Erfindung betrifft ein Tensid-Lösungsmittelgemisch sowie dessen Verwendung zur Beschichtung von Sensoren, beispielsweise von Sensoren, die im Bereich der Koagulationsmessung zum Einsatz kommen.

## Beschreibung

Die Erfindung betrifft die Verwendung eines Tensid-Lösungsmittelgemisches zur Beschichtung von Sensoren, beispielsweise von Sensoren, die im Bereich der Koagulationsmessung zum Einsatz kommen.

Messsysteme oder -sensoren sind in verschiedensten Ausgestaltungen bekannt. Um mit möglichst kleinen Probevolumina zuverlässige Messungen zu erhalten, werden oftmals analytische Testelemente mit Kapillaren eingesetzt, die es ermöglichen, auch geringe Mengen an Probevolumen, typischerweise weniger Microliter, zuverlässig zu dosieren und innerhalb des Testelements zu transportieren. Kapillar-Testelemente können für verschiedenste Nachweise eingesetzt werden und erlauben eine schnelle, einfache und zuverlässige Analyse unter Verwendung von sehr kleinen Testfluidproben. Kapillar-Testelemente werden deshalb bespielsweise insbesondere zur Analyse von Blut oder von anderen biologischen Flüssigkeiten eingesetzt.

Kapillar-Testelemente sind im Allgemeinen so ausgestaltet, dass sie einen Bereich zur Aufnahme des Testfluids, also eine Probenaufgabestelle aufweisen sowie wenigstens einen mit der Probenaufgabenstelle durch eine Kapillare verbundenen Messort. Die Kapillare umfasst eine Kapillaröffnung, die mit der Probenaufgabeöffnung in Verbindung steht, so dass wenn ein Fluid auf das Testelement aufgebracht wird, das Fluid durch die Kapillare zum Messort durch Kapillarwirkung befördert wird. Am Messort können dann die zu detektierenden Parameter in der Probeflüssigkeit bestimmt werden.

Die Kapillare und der Messort werden so ausgestaltet, dass sie eine zuverlässige Analyse mit einem minimalen Testfluidvolumen ermöglichen. In einigen Vorrichtungen sind die Kapillarleitung und der Messort gleicherweise ausgestaltet; haben also insbesondere den gleichen Querschnitt und erscheinen somit als einheitliches Kapillarvolumen. Es ist aber auch möglich, in den Testelementen Kapillarleitung und das Messort-Volumen unterschiedlich auszugestalten.

Das Messort-Volumen enthält typischerweise Komponenten, die mit dem Probenfluid oder Bestandteilen davon wechselwirken, wodurch ein Nachweis von physikalischen oder chemischen Eigenschaften der Probeflüssigkeit oder Bestandteilen davon ermöglicht wird. Der Nachweis kann dabei beispielsweise photometrisch, elektrochemisch, elektrometrisch, akustisch oder mechanisch erfolgen.

Zur Auswertung der Signale des Kapillarteststreifens wird dieser oftmals in Kombination mit einer zweiten Vorrichtung verwendet, typischerweise einem elektronischen Instrument, mit dem Vorhandensein oder das Ausmaß einer Wechselwirkung der Probe oder eines oder mehrerer Analyten in der Probe mit einer oder mehreren Komponenten des Kapillartestelements nachgewiesen werden kann. Mit dem elektronischen Instrument werden solche Wechselwirkungen typischerweise durch photometrische oder elektrometrische Techniken ausgewertet.

In der Anwendung sind Kapillartestelemente häufig zum Einsetzen in das elektronische Instrument vorgesehen, bevor das Testelement mit der fluiden Probe beladen wird. Dabei ragt ein Teil des Testelements aus dem elektronischen Instrument heraus, auf dem dann die Probe aufgebracht wird und das Testergebnis kann unmittelbar am elektronischen Gerät abgelesen werden. Beispielsweise können Koagulationssensoren auf diese Weise ausgestaltet sein.

Während Kapillartestelemente den Vorteil bieten, eine reproduzierbare Analyse von kleinen Probevolumen durchführen zu können, führen die dadurch bedingten kleinen Dimensionen der Kapillaren auch zu Handhabungsschwierigkeiten. Für eine reproduzierbare und präzise Analyse ist nämlich eine schnelle Befüllung der Kapillare erforderlich. Da zudem der Probenauftrag, beispielsweise ein Blutauftrag aus Hygienegründen möglichst weit vom elektronischen Gerät entfernt geschehen soll, ergeben sich oftmals relativ lange Kapillarwege innerhalb der Testdevices.

Eine Aufgabe der vorliegenden Erfindung war es deshalb, eine möglichst kurze und reproduzierbare Füllzeit von solchen Kapillaren mit einem aufzutragenden Probefluid bereitzustellen.

Diese Aufgabe wird erfindungsgemäß gelöst durch einen elektrochemischen Sensor zur Bestimmung eines Gerinnungsparameters, umfassend (i) einen Träger, (ii) mindestens zwei Elektroden, die auf dem Träger aufgebracht sind, (iii) mindestens ein Testreagenz sowie (iv) eine Tensid-enthaltende Beschichtung.

Erfindungsgemäß wurde festgestellt, dass durch Benetzung der Innenoberfläche von Kapillaren mit einem Tensid eine schnelle Befüllung der Kapillare mit einem Probefluid ermöglicht wird. Es wurde dabei festgestellt, dass ein Tensidauftrag möglich ist, bei dem im Vorprozess aufgebrachte Testreagenzien oder andere Komponenten nicht zerstört oder angelöst werden. Erfindungsgemäß wird deshalb eine Tensid-haltige Beschichtung, insbesondere in Form eines Tensid-Lösemittelgemisches in einem eigenen Schritt nach dem Aufbringen von Elektroden und Testreagenzien aufgebracht.

Im Gegensatz zu einer Nachbeschichtung mit einem Tensid, wie sie erfindungsgemäß vorgesehen ist, macht ein Tensidauftrag als Vorbeschichtung die Aufbringung von Testreagenzien aufgrund der Verschlechterung der Oberflächenspannung der Sensorfolie praktisch unmöglich.

Grundsätzlich können zur Aufbringung des Tensids beliebige Tensid-Lösemittelgemische eingesetzt werden. Bevorzugte Tenside sind Octanoyl-N-methylglucamid (Mega8) oder DONS.

Erfindungsgemäß wurde festgestellt, dass besonders gute Ergebnisse bei Einsatz von Octanoyl-N-methylglucamid (Mega8) erhalten werden.

Es wurde insbesondere festgestellt, dass die verwendete Mega8-Lösung ein inertes Verhalten gegen das Trägermaterial, gegen organische Proben, insbesondere Blut, sowie gegen die Testreagenzbeschichtung aufweist und somit die Durchführung des Tests weder negativ beeinflusst noch stört.

Als Lösemittel können Wasser und organische Lösemittel eingesetzt werden, besonders bevorzugt werden Lösemittel auf Basis von Alkohol, bevorzugt von C₁-C₄-Alkohol, und insbesondere auf Basis von Ethanol verwendet. Bevorzugt wird als Lösemittel Alkohol, insbesondere Ethanol eingesetzt mit einem Wassergehalt von ≤ 5 %, insbesondere von ≤ 2 %. Dadurch kann ein Anlösen der zuvor aufgebrachten Testreagenz-Beschichtung beim Aufbringen der Tensid-Lösung vermieden werden.

Besonders bevorzugt ist ein Tensid-Lösemittelgemisch, welches Alkohol, insbesondere Ethanol und Mega8 umfasst, besonders bevorzugt ein Lösemittelgemisch, welches ausschließlich Ethanol und Mega8 enthält.

Der Anteil an Alkohol, und insbesondere an Ethanol im Tensid-Lösungsmittelgemisch, beträgt vorzugsweise ≥ 50 Masseprozent, mehr bevorzugt ≥ 80 Masseprozent, mehr bevorzugt ≥ 90 Masseprozent, noch mehr bevorzugt ≥ 95 Masseprozent, und besonders bevorzugt ≥ 98 Masseprozent und bis zu < 100 Masseprozent, mehr bevorzugt ≤ 99,8 Masseprozent. Der Anteil an Tensid und insbesondere der Anteil an Mega8 beträgt vorzugsweise ≥ 0,1 Masseprozent, mehr bevorzugt ≥ 0,3 Masseprozent bis zu 3 Masseprozent, mehr bevorzugt ≤ 2 Masseprozent, und am meisten bevorzugt ≤ 0,6 Masseprozent, wobei die Masseprozent jeweils auf die Gesamtmasse des Tensid-Lösungsmittelgemisches bezogen sind. Das Tensid-Lösungsmittelgemisch ist vorzugsweise wasserfrei und enthält insbesondere ≤ 0,1 Masseprozent Wasser.

Bevorzugt wird als Lösemittel 98 % Alkohol eingesetzt. Besonders bevorzugt ist ein Tensid-Lösemittelgemisch bestehend aus 98 bis 99,9 Masseprozent Ethanol und 0,1 bis 2 Masseprozent Octanoyl-N-methylglucamid. Kleine Restmengen Wasser sind tolerierbar, z.B. bis zu 5 Masseprozent, insbesondere bis zu 2 Masseprozent.

Das Auftragen des Tensid-Lösungsmittelgemisches auf die Sensoroberfläche und insbesondere auf die Kapillaroberfläche erfolgt erfindungsgemäß bevorzugt mittels eines Piezo-Druckkopfes. Das Funktionsprinzip beruht auf einer Piezo-Kunststofffolie, die sich durch Anlegen einer Spannung schlagartig ausdehnt. Die Pumpkammer des Piezo-Kopfes (Kavität) verändert dadurch ihr Volumen und beschleunigt die Flüssigkeit in der Düse. Es bildet sich ein Tropfen, der mit hoher Geschwindigkeit die Düse verlässt. Die Folie schwingt zurück und der Tropfen reißt ab und wird mit hoher Geschwindigkeit auf die Oberfläche der Sensorfolie geschleudert. In der Piezo-Düse zieht sich der Miniskus der Flüssigkeit zurück, wobei die Oberflächenspannung der Piezo-Kunststofffolie ein Einsaugen von Luft verhindert. Die Flüssigkeit, also das Tensid-Lösungsmittelgemisch, wird dann aus einem Versorgungskanal nachgeführt und steht einer weiteren Dosierung zur Verfügung.

Ein alternatives Auftragsverfahren ist beispielsweise mittels eines Ultraschallzerstäubers.

Es wurde erfindungsgemäß festgestellt, dass insbesondere mittels eines Piezo-Druckkopfes ein homogener und reproduzierbarer Tensidauftrag erreicht werden kann.

Der Träger (i) des Testelements ist bevorzugt ein inerter Träger und wird insbesondere aus einem isolierenden Material gebildet. Geeignete Materialien für den Träger sind insbesondere elektrisch isolierende Kunststoffe, wie beispielsweise Vinylpolymere, Polyimide, Polyester oder Polystyrole, bevorzugt Polyester. Ein besonders geeignetes Polyestermaterial ist beispielsweise eine Melinex-Folie.

Das erfindungsgemäße Testelement weist auf dem Träger weiterhin mindestens zwei Elektroden (ii) auf. Mindestens eine Elektrode ist eine sogenannte Arbeitselektrode. Die Testelemente können lediglich mindestens eine Arbeits- und eine Gegenelektrode aufweisen. Es ist aber auch möglich, eine Referenzelektrode, wie beispielsweise eine Ag/AgCl-Referenzelektrode vorzusehen. Die Elektroden können aus allen gängigen Elektrodenmaterialien aufgebaut sein, beispielsweise aus Metallen, Edelmetallen, Legierungen oder Graphit, vorzugsweise aus Edelmetallen und besonders bevorzugt aus Palladium, Platin oder Gold. In einer besonders bevorzugten Ausführungsform weist das Testelement Elektroden aus Gold auf. Solche Elektrodenstrukturen können beispielsweise durch Laserablation einer Gold-beschichteten Kunststoff-Folie, insbesondere einer Gold-beschichteten Polyester-Folie gebildet werden. Ein solches Laserablationsverfahren ist bespielsweise in WO 01/25775 beschrieben.

Die erfindungsgemäßen Tesstelemente weisen weiterhin wenigstens ein Testreagenz (iii) auf. Die konkrete Ausgestaltung hinsichtlich der Wahl der Materialien und die Anordnung der Reagenzien hängt vom jeweils zu bestimmenden Gerinnungsparameter ab. Bei den sogenannten Globaltests wird beispielsweise die Gerinnungszeit als solche bestimmt. Dies kann über die Bestimmung der Aktivität der Protease Thrombin erfolgen. Solche Tests weisen als Testreagenz ein Thrombinsubstrat auf, welches bei Spaltung durch Thrombin ein elektrochemisch bestimmbares Abspaltprodukt bildet. Durch die Verwendung von anderen Proteasesubstraten können andere Gerinnungstests, beispielsweise ein Anti-Faktor Xa-Test oder die Bestimmung von einzelnen Gerinnungsfaktoren realisiert werden, wobei dann jeweils der Peptidteil des Substrats an die zu bestimmende Protease angepasst wird.

Erfindungsgemäß bevorzugt werden Testreagenzien vorgesehen, mit denen die Prothrombinzeit (PT) die aktivierte Clotting-Zeit (ACT), die aktivierte parzielle Thromboplastinzeit (aPTT), die Ecarin Clotting Time (ECT), der Faktor Xa bestimmt werden kann oder/und mit denen ein Thrombin-Generation Test (TGT) durchgeführt werden kann. Besonders bevorzugt sind Testreagenzien vorgesehen, welche die Durchführung eines Prothrombinzeit-Test erlauben.

Hierfür umfasst das Testreagenz ein Thrombinsubstrat mit einer durch die Protease Thrombin abspaltbaren, elektrochemisch detektierbaren Gruppe, beispielsweise einer Phenylendiamingruppe. Solche Testreagenzien sind beispielsweise in WO 01/63271 beschrieben. Die Testreagenzien werden bevorzugt in Form eines oder mehrerer Reagenzstriche auf das Testelement und insbesondere auf die Elektroden aufgebracht. Es ist auch möglich, die Reagenzien nicht direkt auf die Elektroden, sondern in die Nähe der Elektroden, beispielsweise neben den Elektroden aufzubringen und zu trocknen. In diesem Fall werden die Reagenzien mit der Probe während der Messung zu den Elektroden transportiert. Es ist auch möglich, einzelne Bestandteile der Reagenzien auf unterschiedliche Kompartimente des Testelements aufzubringen.

In einer weiteren bevorzugten Ausführungsform wird zusätzlich zum Testreagenz als weiteres Reagenz eine AgAgCl-Beschichtung aufgebracht.

Der erfindungsgemäße elektrochemische Sensor ist insbesondere ein Kapillar-Sensor. Der Sensor umfasst somit insbesondere einen Kapillarbereich, den die Probe durchläuft. Dieser Kapillarbereich kann beispielsweise eine Verbindung zwischen dem Probeauftragort und einem Messort sein, welche als Kapillare ausgebildet ist. Bevorzugt ist auch der Messort selbst als Kapillare ausgebildet. Die mindestens zwei Elektroden, das mindestens eine Testreagenz sowie die Tensid-enthaltende Beschichtung befinden sich vorzugsweise in einer Kapillare. Es ist auch möglich, den elektrochemischen Sensor so auszugestalten, dass er eine Verbindung zwischen Probeauftrageort und Messort, welcher als Kapillare ausgebildet ist, aufweist und weiterhin der Messort selbst ebenfalls als Kapillare ausgebildet ist, wobei sowohl die Verbindungskapillare als auch der kapillare Messort eine Tensid-enthaltende Beschichtung, wie hierin beschrieben, aufweisen.

Ein wesentlicher Aspekt der Erfindung ist eine Tensid-Beschichtung (iv), die auf das Testreagenz und vorzugsweise zudem auch auf Bereiche des Trägers, welche im Sensor zumindest Teile der Wandung von Kapillarstrukturen bilden, aufgebracht ist. Durch diese Tensid-Beschichtung wird eine schnelle Befüllung der Kapillare ermöglicht. Die Füllzeit der Kapillare ist grundsätzlich von der Kapillargeometrie, von den Oberflächeneigenschaften der Kapillarwände, von den Beschichtungen, insbesondere dem vorhandenen Testreagenz und Elektrodenbestandteilen sowie von der Probe selbst abhängig. Probenparameter, die die Füllgeschwindigkeit beeinflussen, sind beispielsweise die Art der Probe, z.B. Kalibrator, Blut mit hohem oder niedrigem Hämatokrit etc. sowie Probenparameter wie Temperatur, Viskosität usw. Viele dieser Parameter sind je nach Art des durchzuführenden Testes oftmals festgelegt. Durch die erfindungsgemäß vorgesehene zusätzliche Tensid-Beschichtung wird eine reproduzierbar kurze Füllzeit der Kapillare erzielt. Die Füllzeit liegt insbesondere innerhalb der Haltbarkeitsgrenzen des Sensors für alle eingesetzten Probematerialien. Die Füllzeit beträgt insbesondere ≤ 5 s, mehr bevorzugt ≤ 3 s, insbesondere < 2 s.

Bevorzugt weist die Kapillarstruktur eine Länge von ≥ 1 mm, bevorzugt ≥ 5 mm, mehr bevorzugt ≥ 10 mm, und insbesondere ≥ 12 mm auf. Der Querschnitt der Kapillaren beträgt als Innendurchmesser vorzugsweise ≤ 2 mm, mehr bevorzugt ≤ 1 mm, noch mehr bevorzugt ≤ 500 µm und am meisten bevorzugt ≤ 300 µm. Die Kapillare kann aber auch einen anderen Querschnitt aufweisen, beispielsweise einen rechteckigen Querschnitt, wobei hier Größen mit einer Breite von ≤ 2 mm, bevorzugt ≤ 1,5 mm und Höhen von ≤ 1 mm, bevorzugt ≤ 500 µm, mehr bevorzugt ≤ 200 µm geeignet sind.

Erfindungswesentlich ist, dass die Tensid-Beschichtung auf das Testreagenz aufgebracht ist und nicht etwa vor der Beschichtung mit den Testreagenzien oder als Teil der Testreagenzienlösung aufgetragen wird. Ein Vorauftrag von Tensid macht eine Beschichtung mit Testreagenz unmöglich. Im Gegensatz zu Vorgehensweisen, bei denen das Tensid als Bestandteil der Testreagenzienlösung eingesetzt wird, wird erfindungsgemäß der Vorteil erhalten, dass der Testreagenzienbereich deutlich besser benetzbar ist. Da, wie erfindungsgemäß festgestellt, eine Nachbeschichtung von Testreagenzbereichen nicht zu einer Anlösung der aufgebrachten Testreagenzienmischung führt, resultiert das Aufbringen von Tensid darin, dass das Tensid an der Oberfläche verbleibt und somit zu einer Hydrophilisierung der Oberfläche führt. Im Gegensatz dazu ist beim Aufbringen von Tensid als Bestandteil oder zusammen mit der Textreagenzienlösung das Tensid wie auch die anderen Stoffe des Testreagenziengemisches zunächst überall in der Lösung (und nicht nur an der später entstehenden Oberfläche) verteilt. Dies führt dazu, dass beim Trocknen das Tensid durchgängig durch die ganze Testreagenzienschicht verteilt ist und sich nicht in größerem Maße an der Oberfläche anlagert, an der die eigentliche Tensidwirkung benötigt wird. Somit bietet ein zusammen mit dem Testreagenz aufgebrachtes Tensid keine Hydrophilierung der Oberfläche und somit keine Verbesserung der Benetzbarkeit. Weiterhin sind in vielen Testreagenzien, insbesondere in PT-Reagenzien, Filmbildner enthalten. Solche Filmbildner haben aber die Eigenschaft, an die Oberfläche zu wandern und den Testreagenzfilm abzudecken. Dies verhindert weiterhin eine Wirkung von im Testreagenzgemisch enthaltenem Tensid zur Verbesserung der Benetzbarkeit.

Weiterhin kann erfindungsgemäß eine Tensidbeschichtung über verschiedene oder den gesamten Bereich der Kapillarinnenfläche erreicht werden, so dass die Tensid-enthaltende Beschichtung sowohl auf den inerten Bereichen des Trägers als auch auf den Elektrodenstrukturen und auch auf dem Testreagenz als Beschichtung aufgebracht ist. Die erfindungsgemäßen Vorteile werden nur durch das nachträgliche Aufbringen einer Tensid-Beschichtung auf die zuvor aufgetragene Testreagenz-Beschichtung erreicht.

Erfindungsgemäß liegt die Tensid-enthaltende Beschichtung homogen und vorzugsweise auch vollflächig auf der gesamten Innenwandung des elektrochemischen Sensors, und insbesondere auf der Kapillarinnenwandung des elektrochemischen Sensors vor. Die Beschichtung ist insbesondere vollflächig, zumindest über dem Testreagenz und bevorzugt auch über dem Trägermaterial und über den Elektrodenmaterialien, gegebenenfalls einschließlich AgAgCl. Vorzugsweise wird mit der Tensid-enthaltenden Beschichtung ≥ 10 %, mehr bevorzugt ≥ 50 %, mehr bevorzugt ≥ 80 % und noch mehr bevorzugt ≥ 90 % und am meisten bevorzugt ≥ 99 % der Gesamtfläche gebildet aus Trägeroberfläche, Elektrodenoberflächen sowie Testreagenzoberflächen bedeckt.

Die Tensid-enthaltende Beschichtung wird vorzugsweise auf eine Sensorfolie aufgebracht, aus der in weiteren Schritten dann ein elektrochemischer Sensor gebildet wird.

Es wurde überraschenderweise festgestellt, dass durch den Auftrag eines Tensid-Lösungsmittelgemisches eine gleichmäßige Tensid-Beschichtung erreicht werden kann. Insbesondere werden sowohl beschichtete und mit Testreagenz versehene Bereiche als auch nicht beschichtete Bereiche der Sensorfolie, die dann die Füllkapillare des resultierenden Testelements bilden, homogen und stabil mit dem Tensid-Lösungsmittelgemisch benetzt. Zudem erfolgt die Benetzung unter Beibehaltung der physikalischen Stabilität von zuvor beschichteten Reagenzien, insbesondere Testreagenzien oder/und Elektrodenbestandteilen, wie beispielsweise einem PT-Testreagenz oder AgAgCl. Die zuvor beschichteten und getrockneten Reagenzien, insbesondere PT-Testreagenz und AgAgCl, werden durch den nachfolgenden Prozessschritt des Tensidauftrags nicht wieder angelöst. Form und Lage der Beschichtungen bleiben somit unverändert.

Der Tensidauftrag erfolgt bevorzugt unter Verwendung eines Piezo-Druckkopfes. Insbesondere unter Verwendung eines Piezo-Druckkopfes kann eine homogene und reproduzierbare Beschichtung erhalten werden. Die Erfindung betrifft deshalb auch ein Verfahren zur Beschichtung einer Sensorfolie mit einem Tensid-Lösemittelgemisch, welches dadurch gekennzeichnet ist, dass das Auftragen des Tensid-Lösungsmittelgemisches durch Piezo-Drucken bewerkstelligt wird.

Bevorzugt wird das Tensid-Lösemittelgemisch kontinuierlich aufgebracht. Besonders günstig ist beispielsweise ein kontinuierliches Verfahren zur gezielten lokalen und diskreten Applikation eines Tensid-Lösungsmittelgemisches auf vorbeschichtete Sensorfolienbahnen, wobei die Sensorfolien insbesondere eine Gold-beschichtete, Laser-ablatierte Polyesterfolie sind, welche zuvor in einem atmosphärischen Plasma-Verfahren behandelt wurde. Es wurde festgestellt, dass trotz der unterschiedlichen Oberflächenspannungen einer solchen Mischfläche eine gleichmäßige und homogene Benetzung mit Tensid erhalten werden kann.

Weiterhin liefert das erfindungsgemäße Auftragen des Lösemittelgemischs auf die Sensorfolie eine gute Verarbeitbarkeit, insbesondere Bevorratung, Förderung und Auftragung in der Produktion auch unter Arbeitssicherheitstechnischen Aspekten. Weiterhin ist, insbesondere bei Verwendung von Alkoholen und bevorzugt Ethanol eine schnelle Trocknung nach Auftrag zu erzielen. Die Beschichtung kann als voll integrierter Auftragprozessschritt in den gesamten Herstellungsprozess des Testelements eingefügt werden.

Es kann somit als kontinuierliches Verfahren zum InLine-Auftrag von Tensid auf eine beschichtete Sensorfolie, insbesondere auf eine mit Elektroden und Testreagenzien beschichtete Sensorfolie ausgestaltet sein.

Der Piezo-Druckkopf ist vorzugsweise als Bestandteil des gesamten Auftragssystems ausgestaltet. Das Auftragssystem besteht im Wesentlichen aus einem Steuergerät, einem Fluid-System, insbesondere zur Bevorratung und zur Durchflussmessung, und dem Piezo-Auftragskopf. Der erzielte flächige Auftrag an Tensid ist im Allgemeinen abhängig von den Parametern, Bahngeschwindigkeit, Anstellwinkel der Druckleiste zur Sensorfolie, welcher die Auftragsbreite bestimmt, sowie dem Abstand des Auftragskopfes zur zu beschichtenden Sensorfolie. Weiterhin wichtig für die Wirkungsweise des Tensidauftrags ist der diskret benetzte Bereich, insbesondere Abschnitte bestehend aus Sensorfolien bzw. Kapillarmaterial, Testreagenz, insbesondere PT-Testreagenz, und Elektrodenmaterial, wie beispielsweise Gold oder AgAgCl, die Tropfengröße und das Tropfenbild auf dem Auftragsbereich. Weiterhin von Bedeutung ist die Art und Menge des Tensids sowie des Lösungsmittels. Daraus resultiert die aufgetragene Menge Tensid auf dem Teststreifen.

Die Auftragsleistung kann beispielsweise durch Synchronisierung des Piezo-Auftragskopfes mit der Bahnförderung der Maschine, also der Maschinengeschwindigkeit festgelegt und automatisch angepasst werden. Bevorzugt beträgt die Bahngeschwindigkeit 0 bis 50 m/min, insbesondere 0 bis 25 m/min. Der Anstellwinkel der Druckleiste zur Sensorfolie beträgt vorzugsweise 8° bis 20°, insbesondere 11° bis 13°, und besonders bevorzugt 12°, wobei 12° einer Auftragsbreite von 18 mm entsprechen. Der Abstand des Auftragskopfes zur zu beschichtenden Sensorfolie beträgt vorzugsweise 2 mm bis 10 mm, insbesondere 4 mm bis 8 mm. Die Auftragsleistung wird vorzugsweise in einen Bereich von 100 bis 1000 µl/min, insbesondere 200 bis 750 µl/min, eingestellt.

Die Beschichtung mit Tensid-Lösemittelgemisch erfolgt vorzugsweise InLine bei der Herstellung des Testelements innerhalb des Teilprozesses Beschichtung und Trocknung.

Nach dem Tensidauftrag erfolgt, vorzugsweise ebenfalls InLine, eine Trocknung des Lösemittels, beispielsweise eine schonende Trocknung mittels Heizkanal und mittels Heizplatten. Durch die Verwendung eines alkoholischen und insbesondere eines ethanolischen Lösungsmittels reduziert sich die Trocknungsstrecke sowie die Trocknungstemperatur verglichen zur Verwendung von Wasser als Lösungsmittel. Dies führt zu Vorteilen bezüglich der Temperaturbelastung der zuvor aufgetragenen Testreagenzien, beispielsweise der PT-Testreagenzien. Mit den erfindungsgemäßen Sensoren wird insbesondere eine reproduzierbare und kurze Füllzeit der Kapillare realisiert. Weiterhin wurde festgestellt, dass durch den Tensidauftrag und insbesondere bei Verwendung des Tensids Mega8 keine negative Beeinflussung der Testperformance zu beobachten ist. So wird insbesondere die Gerinnungszeit, also der zu messende Testparameter, nicht beeinflusst und die Probematerialien werden nicht negativ beeinflusst oder zerstört. Insbesondere wurde eine Füllzeit des Devices innerhalb der Haltbarkeitsgrenzen des Sensors für alle enthaltenen Probematerialien erreicht.

Das hierin beschriebene Verfahren zur Herstellung eines elektrochemischen Sensors ist ebenfalls Gegenstand der Erfindung. Die Erfindung umfasst somit insbesondere auch ein Verfahren zur Herstellung eines elektrochemischen Sensors umfassend die Schritte
(a) Bereitstellen eines Trägers,
(b) Aufbringen von mindestens zwei Elektroden auf den Träger,
(c) Aufbringen mindestens eines Testreagenzes,
(d) nach Schritt (c) Aufbringen eines Tensid-Lösungsmittelgemisches, und
(e) Anbringen eines Deckels, wodurch ein Kapillar-Sensorelement gebildet wird sowie optional
(f) Vereinzeln der Sensorelemente.

Der Träger, insbesondere ein inerter Träger wird vorzugsweise zunächst als Trägerfolie bereitgestellt, beispielsweise als Polyesterfolie. Auf diese Trägerfolie werden dann zunächst Elektrodenstrukturen aufgebracht, beispielsweise Goldelektrodenstrukturen. In einem weiteren Schritt werden Reagenzien, wie etwa AgAgCl sowie Testreagenzien, beispielsweise PT-Testreagenzien, auf bestimmte Bereiche der Trägerfolie aufgebracht. Die Reagenzbeschichtung wird vorzugsweise vor einer Weiterverarbeitung getrocknet. Nach Schritt (c), also nach dem Aufbringen mindestens eines Testreagenzes, wird erfindungsgemäß ein Tensid-Lösungsmittelgemisch aufgebracht. Vorzugsweise erfolgt das Aufbringen eines Tensid-Lösungsmittelgemisches erst, nachdem sowohl die Elektroden (Schritt (b)) als auch das Testreagenz (Schritt (c)) aufgebracht worden sind. Das Aufbringen erfolgt vorzugsweise mittels eines Piezo-Druckkopfes. Bei dem Tensid-Lösungsmittelgemisch handelt es sich vorzugsweise um eine alkoholische Mega8-Lösung. Nach dem Aufbringen des Tensid-Lösungsmittelgemisches erfolgt vorzugsweise ein Trocknungsschritt. Anschließend wird ein Deckel auf der Sensorfolie aufgebracht, beispielsweise auflaminiert, und anschließend können aus der Sensorfolie die elektrochemischen Sensoren geschnitten werden.

Die Erfindung betrifft weiterhin ein elektrochemisches Testelement-Analysesystem, umfassend als Testelement wenigstens einen elektrochemischen Sensor, wie hierin beschrieben, sowie wenigstens ein Strom- oder Spannungsmessgerät zur Auswertung des Sensors.

Die Erfindung wird durch die beigefügten Figuren und die nachfolgenden Beispiele weiter erläutert.

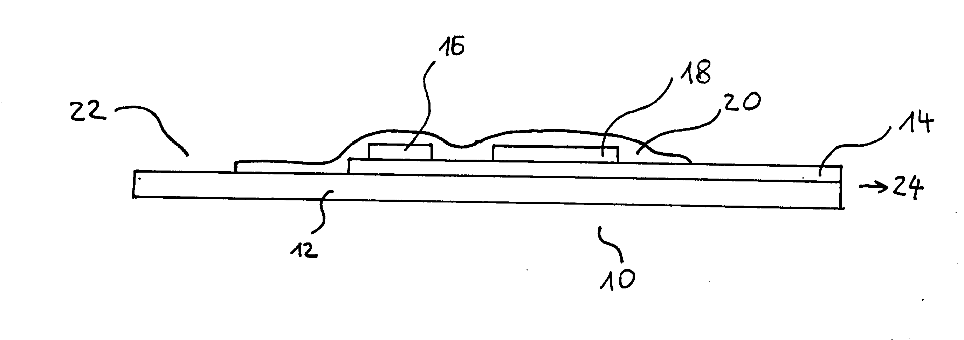

**Figur 1** zeigt schematisch den Aufbau einer Sensorfolie, umfassend ein Trägermaterial, zwei Goldelektroden, darauf beschichtete Testreagenzien sowie eine darauf aufgebrachte Tensid-haltige Beschichtung. Die Sensorfolie (10) umfasst ein Trägermaterial (12) auf das Goldelektrodenstrukturen (14) aufgebracht sind. Auf die Goldelektrodenstrukturen können Reagenzien (16), z.B. Ag/AgCl aufgebracht sein sowie Testreagenzien (18), beispielsweise PT-Testreagenzien. Auf die insbesondere mit den Testreagenzien vorbeschichtete Sensorfolie wird dann eine Tensid-haltige Beschichtung (20) aufgebracht. Weiterhin angedeutet sind ein Bereich zur Probenauftragung (22) sowie ein Anschluss an ein Messgerät (24).

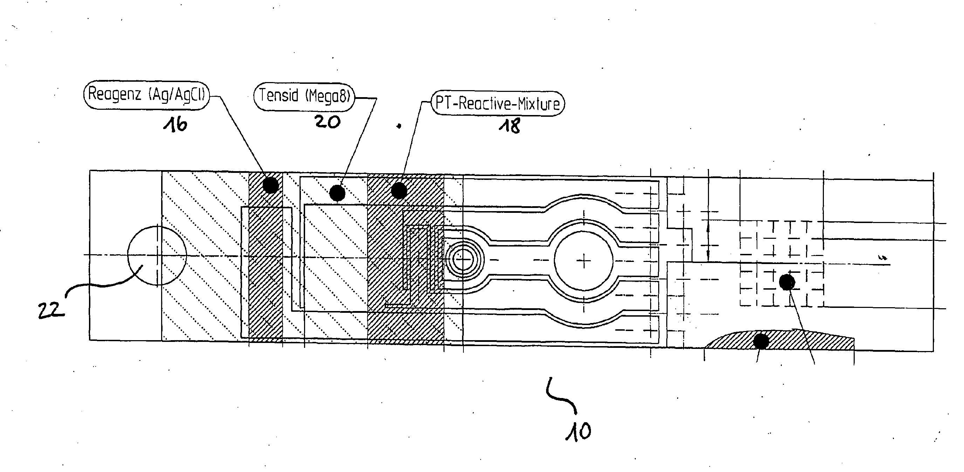

**Figur 2** zeigt einen elektrochemischen Sensor, gebildet unter Verwendung einer erfindungsgemäß hergestellten Sensorfolie.

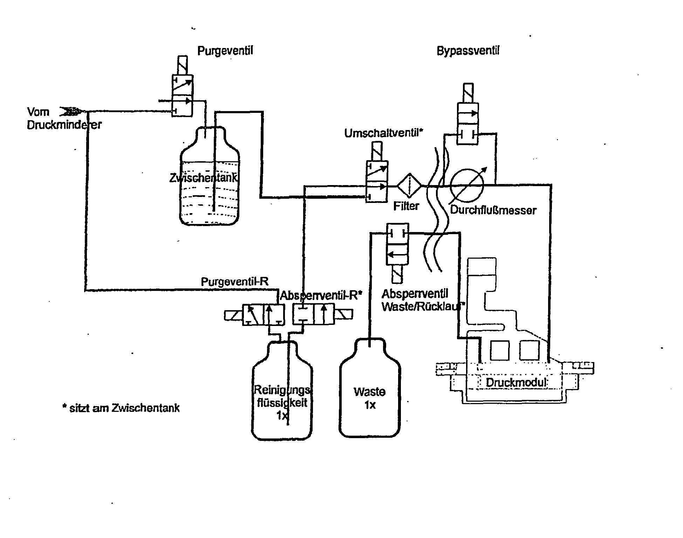

**Figur 3** zeigt schematisch ein Auftragesystem umfassend einen Piezo-Druckkopf.

### Beispiele

### Beispiel 1

Eine Gold-beschichtete Laser-ablatierte Polyesterfolie wurde mit einem atmosphärischen Plasma-Verfahren behandelt. Anschließend wurden PT-Testreagenzien sowie AgAgCl aufgebracht und getrocknet. Nachfolgend wurde InLine ein Mega8-Ethanol-Gemisch mit einer Tensidkonzentration von 0,5 Masseprozent lnLine mittels eines Piezo-Druckkopfes aufgebracht. Die Auftragsleistung betrug 86 bis 458 µl/min bei 8 m/min. Es wurde eine gleichmäßige homogene Beschichtung mit Netzmittel über eine Breite von 18 mm +/- 1 mm vollflächig über das PT-Reagenzgemisch, AgAgCl sowie das Sensorfolienmaterial erzielt.

### Beispiel 2

Eine Gold-beschichtete Laser-ablatierte Polyesterfolie wurde mit einem atmosphärischen Plasma-Verfahren behandelt. Anschließend wurden PT-Testreagenzien sowie AgAgCl aufgebracht und getrocknet. Nachfolgend wurde InLine ein Mega8-Ethanol-Gemisch mit einer Tensidkonzentration von 0,25; 0,375; 0,5 bzw. 0,75 Masseprozent InLine mittels eines Piezo-Druckkopfes aufgebracht. Die Auftragsleistung betrug 160 µl/min bei 8 m/min. Es wurde eine gleichmäßige homogene Beschichtung mit Netzmittel über eine Breite von 18 mm +/- 1 mm vollflächig über das PT-Reagenzgemisch, AgAgCl sowie das Sensorfolienmaterial erzielt.

## Patentansprüche

1. Elektrochemischer Sensor zur Bestimmung eines
Gerinnungsparameters, umfassend
(i) einen Träger,
(ii) mindestens zwei Elektroden, die auf dem Träger aufgebracht sind,
(iii) mindestens ein Testreagenz sowie
(iv) eine Tensid-enthaltende Beschichtung.

2. Sensor nach Anspruch 1,
**dadurch gekennzeichnet,**
**dass** die Tensid-Beschichtung (iv) Octanoyl-N-methylglucamid umfasst.

3. Sensor nach Anspruch 1 oder 2,
**dadurch gekennzeichnet,**
**dass** das Testreagenz ein Prothrombin-Testreagenz ist.

4. Sensor nach einem der Ansprüche 1 bis 3,
**dadurch gekennzeichnet,**
**dass** die Tensidbeschichtung (iv) durch Piezo-Drucken aufgebracht ist.

5. Sensor nach einem der Ansprüche 1 bis 4,
**dadurch gekennzeichnet,**
**dass** er weiterhin umfasst
(v) einen Probeauftrageort,
(vi) einen Messort sowie
(vii) eine Verbindung zwischen Probeauftrageort und Messort, welche als Kapillare ausgebildet ist.

6. Verfahren zur Beschichtung einer Sensorfolie mit einem Tensid-Lösungsmittelgemisch,
**dadurch gekennzeichnet,**
**dass** das Auftragen des Tensid-Lösungsmittelgemisches durch Piezo-Drucken bewerkstelligt wird.

7. Verfahren nach Anspruch 6,
**dadurch gekennzeichnet,**
**dass** das Tensid-Lösemittelgemisch Octanoyl-N-methylglucamid als Tensid und Ethanol als Lösemittel umfasst.

8. Verfahren nach Anspruch 6 oder 7,
**dadurch gekennzeichnet,**
**dass** das Piezo-Drucken bei einer Bahngeschwindigkeit der Sensorfolie von 0 bis 25 m/min, einem Abstand des Piezo-Druckauftragskopfes zur Sensorfolie von 4 mm bis 8 mm und einer Auftragsleistung von 100 bis 750 µl/min durchgeführt wird.

9. Verfahren nach Anspruch 6 bis 8,
**dadurch gekennzeichnet,**
**dass** die Sensorfolie einen inerten Träger, Elektroden sowie ein Testreagenz aufweist.

10. Verfahren zur Bestimmung eines Gerinnungsparameters unter Verwendung eines elektrochemischen Sensors nach einem der Ansprüche 1 bis 5.

11. Verfahren zur Herstellung eines elektrochemischen Sensors umfassend die Schritte
(a) Bereitstellen eines Trägers,
(b) Aufbringen von mindestens zwei Elektroden auf den Träger,
(c) Aufbringen mindestens eines Testreagenzes,
(d) nach Schritt (c) Aufbringen eines Tensid-Lösungsmittelgemisches, und
(e) Anbringen eines Deckels, wodurch ein Kapillar-Sensorelement gebildet wird.

12. Elektrochemisches Testelement-Analysesystem
umfassend
wenigstens einen Sensor nach einem der Ansprüche 1 bis 5, und wenigstens ein Strom- oder Spannungsmessgerät.
